# EUROPEAN PATENT SPECIFICATION

(11) **EP 4 176 872 B1**
(45) Date of publication and mention of the grant of the patent: **10.06.2026**
(21) Application number: 22205248.2
(22) Date of filing: 03.11.2022
(51) Int. Cl.: A61K 31/02, A61K 31/08, A61K 31/19, A61K 31/195, A61K 31/196, A61K 31/202, A61K 31/428, A61K 31/55, A61K 31/7105, A61P 3/04, A61K 31/381, A61K 31/18, A61K 48/00, A61K 49/00, A01K 67/0275, C12N 15/00, C07K 14/47, G01N 33/50

(54) **COMPOSITION COMPRISING A TREK1 ACTIVATOR FOR USE IN A METHOD OF CONTROLLING OBESITY BY REGULATING ACTIVITY OF TREK1**
ZUSAMMENSETZUNG AUFWEISEND EINEN TREK1-AKTIVATOR ZUR VERWENDUNG IN EINEM VERFAHREN ZUR KONTROLLE VON ADIPOSITAS DURCH REGULIERUNG DER AKTIVITÄT VON TREK1
COMPOSITION COMPRENANT UN ACTIVATEUR DE TREK1 POUR SON UTILISATION DANS UN PROCÉDÉ DE LUTTE CONTRE L'OBÉSITÉ PAR RÉGULATION DE L'ACTIVITÉ DE TREK1

(30) Priority: 03.11.2021 KR 20210149971; 01.08.2022 KR 20220095595
(43) Date of publication of application: 10.05.2023
(73) Proprietor: Korea Institute of Science and Technology, Seoul 02792 (KR); Korea Food Research Institute, Wanju-gun, Jeollabuk-do 55365 (KR)
(72) Inventor: HWANG, Eun Mi, 02792 Seoul (KR); KIM, Ajung, 02792 Seoul (KR); LEE, Jaekwang, 54860 Jeollabuk-do (KR); JUNG, Seoyoung, 41239 Daegu (KR)
(74) Representative: advotec.

(56) References cited:
- WO-A1-2005/074923
- DJILLANI ALAEDDINE ET AL: "Role of TREK-1 in Health and Disease, Focus on the Central Nervous System", FRONTIERS IN PHARMACOLOGY, vol. 10, 11 April 2019 (2019-04-11), XP093029887, DOI: 10.3389/fphar.2019.00379
- JAKAB JELENA ET AL: "Adipogenesis as a Potential Anti-Obesity Target: A Review of Pharmacological Treatment and Natural Products", vol. Volume 14, 1 January 2021 (2021-01-01), pages 67 - 83, XP093030925, Retrieved from the Internet <URL:https://www.dovepress.com/getfile.php?fileID=65512> DOI: 10.2147/DMSO.S281186
- SULEIMAN JOSEPH ET AL: "A systematic review on different models of inducing obesity in animals: Advantages and limitations", JOURNAL OF ADVANCED VETERINARY AND ANIMAL RESEARCH, vol. 7, no. 1, 1 January 2020 (2020-01-01), pages 103, XP093031972, DOI: 10.5455/javar.2020.g399

## Description

### [Technical Field]

The present invention relates to obesity control according to the regulation of activity of TREK1.

### [Background Art]

Obesity is very common among modern people and is known as a disease that causes various lifestyle diseases. Accordingly, research on new anti-obesity substances is being actively conducted (Korean Patent No. 10-1662062), but effective treatment methods or therapeutic agents are still somewhat insufficient.

In addition, since the major anti-obesity drugs used so far act as a mechanism to suppress appetite by acting on the central nervous system or the peripheral nervous system, various side effects occur when these drugs are taken for a long period of time due to the mechanism of action, and thus are known to have low drug compliance. Therefore, there is a need for a new obesity treatment strategy with a mechanism that suppresses the division and differentiation of adipocytes rather than a method of suppressing appetite.

In the publication of WO 2005/074923 A1 a method for modulating the sensation of satiety perception and agents useful in modulating, controlling or otherwise affecting inter alia obesity in a subject are disclosed. Thus, mechanosensitive potassium channels, for example, TREK1, KCNK2, as target for the modulation of the perception of satiety are identified.

The publication of Djillani Alaeddine ET AL: "Role of TREK-1 in Health and Disease, Focus on the Central Nervous System", Frontiers in Pharmacology, vol. 10, 11 April 2019, describes the role of TREK1 in health and disease, for example in the nervous system, and describes a list of TREK1 activators.

The publication of Jakab Jelena ET AL: "Adipogenesis as a Potential Anti-Obesity Target: A Review of Pharmacological Treatment and Natural Products", Diabetes, Metabolic Syndrome and Obesity: Targets and Therapy, vol. Volume 14, 1 January 2021, describes that identifying potential adipogenic molecular targets susceptible to modulation by external factors can utilize adipogenesis regulation to control or reverse obesity.

The publication of SULEIMAN JOSEPH ET AL: "A systematic review on different models of inducing obesity in animals: Advantages and limitations", JOURNAL OF ADVANCED VETERINARY AND ANIMAL RESEARCH, vol. 7, no. 1, 1 January 2020, describes several annimal models used for the study of metabolic disease like obesity, with their advantages and limitations.

### [Disclosure]

### [Technical Problem]

One object of the present invention provides a pharmaceutical composition for use in preventing or treating obesity, including a TREK1 activator, which is a substance that increases the activity of TREK1 protein and/or the expression of a gene encoding TREK1 protein, as an active ingredient.

Another object of the present invention provides a composition for use in a method for preventing or treating obesity, the composition comprising a TREK1 activator as active ingredient, the method including enhancing the activity of TREK1, wherein the enhancement of the TREK1 activity includes a treatment with a TREK1 activator and wherein the enhancement of TREK1 is achieved several steps described elsewhere.

Even another object of the present invention provides an in vitro method for screening a preventive or therapeutic agent for obesity, including:
(a) administering a candidate substance for preventing or treating obesity into preadipocyte cells isolated from a subject; and (b) measuring the level of TREK1 activity in the cells of step (a); and (c) selecting the candidate substance as a substance for preventing or treating obesity, when the TREK1 activity level is increased as a result of the measurement in step (b).

The present invention is defined in the appended claims. Any references in the description to methods of treatment refer to the compounds, pharmaceutical compositions, and medicaments of the present invention for use in a method for treatment of the human (or animal) body by therapy.

### [Technical Solution]

The present invention will be described in detail as follows. Meanwhile, each description and embodiment disclosed herein can be applied to other descriptions and embodiments, respectively. Additionally, a number of papers and patent documents have been cited throughout the present specification.

The present inventors confirmed the phenomenon that the potassium ion conductivity is decreased and the membrane potential is depolarized in the process of differentiation into adipocytes, and it was first identified that TREK1 is specifically involved in this process. Therefore, it was confirmed that obesity was induced by promoting differentiation into adipocytes according to inhibition of TREK1, and differentiation of adipocytes was inhibited according to increase in TREK1 activity.

It is another aspect of the present invention to provide a pharmaceutical composition for use in preventing or treating obesity, including a TREK1 activator, which is a substance that increases the activity of TREK1 protein and/or the expression of a gene encoding TREK1 protein, as an active ingredient.

As used herein, the "TREK1" is also designated as "KCNK2", and referred to as "K2p2.1", "TPKC1", "hTREK-1c", "hTREK-1e", "potassium two pore domain channel subfamily K member 2", etc. TREK1 is known to mainly transmit electrical signals in mammalian neurons or to be involved in roles such as ischemia and anesthesia. Gene or protein sequence information of TREK1 can be found in Uniprot O95069, NP_001017424, NP_001017425, NP_055032, *etc.,* and the gene encoding TREK1 may be referred to as *TREK1, KCNK2, etc.*

As used herein, the "TREK1 activator" includes, without limitation, substances capable of enhancing the activity of TREK1. Such activity enhancement includes not only enhancing the activity of the protein, but also increasing the gene expression level or increasing the copy number of the gene.

Examples of substances known to increase the activity of TREK1 are disclosed in Djillani, A., Mazella, J., Heurteaux, C., and Borsotto, M. (2019) Role of TREK-1 in Health and Disease, Focus on the Central Nervous System. Front. Pharmacol. 10:379, etc*.*

In one embodiment, the TREK1 activator may be selected from riluzole, lithium, gabapentin, valproate, carbamazepine, ML67, ML67-33, ML335, ML402, arachidonic acid, chloroform, halothane, isoflurane, diethyl ether, fenamate, flufenamic acid, BL-1249, RNE28, GI-530159, and PUFA.

In another embodiment, the TREK1 activator may be a substance that increases the expression of a gene encoding TREK1. For example, the TREK1 activator may be an expression cassette containing the cDNA of the gene encoding TREK1, and as a specific example, it may be a vector containing the cDNA of the gene encoding TREK1.

As used herein, the "expression cassette" refers to a unit cassette which includes a promoter and a target gene and thus can express the target gene operably linked to the promoter. For the purpose of the present invention, the target gene may be a gene encoding TREK1. The expression cassette may include various factors that can assist efficient expression of the target gene, inside or outside of the expression cassette. The expression cassette may conventionally include a transcription termination signal, a ribosome-binding domain, and a translation termination signal, in addition to the promoter operably linked to the target gene, and may also include enhancer sequences, introns, translation initiation sequences and untranslated regions (UTR), *etc.,* but is not limited thereto.

As used herein, the term "operably linked" means that the promoter sequences capable of initiating and mediating the transcription of the target gene and the gene sequence are functionally linked.

As used herein, the term "vector" refers to a DNA construct containing the polynucleotide sequence encoding the target polypeptide or protein operably linked to a suitable regulatory sequence so as to be able to express the target polypeptide or protein. For the purpose of the present invention, the target protein may be TREK1.

Once transformed into a suitable host cell, the vector may replicate or function independently from the host genome, or may integrate into genome thereof. The vector that can be used in the present invention is not particularly limited, and in one example, plasmids, viruses or bacteriophages may be used, and examples thereof include adeno-associated viruses, *etc.*

In any one of the above-described embodiments, the promoter included in the vector or expression cassette may be specifically expressed in fat cells, for example, adipocytes, but is not limited thereto.

As used herein the "obesity" refers to a state in which excess adipose tissues are in the body. Obesity is also caused by the differentiation of adipocytes, thereby increasing its number, and is usually accompanied by overweight. Obesity is a metabolic disease that affects the entire body, increasing the risk of diabetes, hyperlipidemia, sexual dysfunction, arthritis, and cardiovascular disease, and is also associated with occurrence of cancer in some cases. In addition, since obesity is closely related to various metabolic syndromes including non-alcoholic liver disease, the pharmaceutical composition of the present invention can also be used for the prevention and treatment of metabolic syndrome.

The pharmaceutical composition of the present invention may be administered to an obese patient, and the target obese patient may be typically determined according to the body mass index (BMI) index. In the present invention, BMI means a value obtained by dividing weight (kg) by the square of height (m), and the obese patient of the present invention is defined as an obese patient when the BMI index is 23 kg/m², 24 kg/m², 25 kg/m², 26 kg/m², 27 kg/m², 28 kg/m², 29 kg/m², 30 kg/m², 31 kg/m², 32 kg/m², 33 kg/m², 34 kg/m², 35 kg/m², 36 kg/m², 37 kg/m², 38 kg/m², 39 kg/m², or 40 kg/m² or more. The pharmaceutical composition of the present invention may exhibit effects such as weight loss, reduction in fat mass, *etc.* in obese patients.

As used herein, the term "prevention" refers to all activities that inhibit or delay the occurrence of obesity by administering the pharmaceutical composition, and the term "treatment" refers to all activities that improve or advantageously change the symptoms of obesity by administering the pharmaceutical composition.

The pharmaceutical composition of the present invention may further contain a pharmaceutically acceptable carrier, excipient, or diluent. The pharmaceutically acceptable carrier, excipient, or diluent may be one that is non-naturally occurring.

As used herein, the term "pharmaceutically acceptable" refers to the properties of having a sufficient amount to show a therapeutic effect and not causing adverse effects, and may be easily determined by those skilled in the art based on the factors well known in the medical field, such as the kind of disease, age, body weight, health status, sex, drug sensitivity of a patient, administration route, administration method, administration frequency, duration of treatment, a drug(s) to be mixed or administered simultaneously, *etc.*

The pharmaceutically acceptable excipient may include, but is not particularly limited to, for injections, a buffering agent, a preservative, an analgesic, a solubilizing agent, an isotonic agent, a stabilizing agent, etc., which may be used in combination; and for topical administrations, a base, an excipient, a lubricant, a preservative, *etc.*

The formulation type of the composition of the present invention may be prepared variously by being combined with a pharmaceutically acceptable carrier described above. For example, for injections, the composition may be formulated into unit-dose ampoules or multi-dose forms. The composition may also be formulated into solutions, suspensions, tablets, pills, capsules, sustained-release formulations, *etc.*

Examples of suitable carriers, excipients, and diluents for formulations may include lactose, dextrose, sucrose, sorbitol, mannitol, xylitol, erythritol, maltitol, starch, acacia, alginate, gelatin, calcium phosphate, calcium silicate, cellulose, methyl cellulose, microcrystalline cellulose, polyvinylpyrrolidone, water, methyl hydroxybenzoate, propyl hydroxybenzoate, talc, magnesium stearate, mineral oil, *etc.* Additionally, the composition may further contain a filler, an anti-coagulant, a lubricant, a humectant, a fragrance, a preservative, etc.

Additionally, the composition may be formulated into a preparation of a unit dosage form suitable for the administration into a patient's body, and may be formulated into a preparation useful for protein drugs according to the conventional method in the pharmaceutical field so as to be administered by, for example, parenteral route. In one embodiment, the composition may be injected into adipose tissue.

In one embodiment, the pharmaceutical composition including the TREK1 activator of the present invention may include any pharmaceutically acceptable salt or any other compound capable of providing, directly or indirectly, a biologically active metabolite or residue thereof upon administration to an animal, including a human.

It is still another aspect of the present invention to provide a composition for use in a method for preventing or treating obesity, the composition comprising a TREK1 activator as active ingredient, the method including: enhancing the activity of TREK1. The enhancement of the TREK1 activity includes treatment with a TREK1 activator. The TREK1 activator is as described above. In any one of the above-described embodiments, the enhancement of the TREK1 activity may be enhancing TREK1 activity of adipocytes.

The enhancement of the TREK1 activity is achieved by:
1) increasing the intracellular copy number of a polynucleotide encoding TREK1;
2) modifying the expression regulatory region of a gene encoding TREK1 with a sequence having a strong activity;
3) modifying the nucleotide sequence encoding the 5'-UTR or 3'-UTR of the gene transcript encoding TREK1;
4) modifying the amino acid sequence or the polynucleotide sequence encoding the same such that the activity of TREK1 is enhanced;
5) regulating the transcription or translation such that the activity of TREK1 is enhanced;
6) introducing a foreign polypeptide exhibiting the activity of TREK1 or a foreign polynucleotide encoding the polypeptide (not part of the invention);
7) codon optimization of the polynucleotide encoding TREK1;
8) post-translational modification such that the activity of TREK1 is enhanced; or
9) a combination of two or more selected from above 1) to 5, 7 or 8).

The 1) method of increasing the intracellular copy number of a polynucleotide encoding TREK1 may be achieved by introducing a vector, which is operably linked to the polynucleotide encoding the polypeptide and is able to replicate and function regardless of a host cell, into the host cell. Alternatively, the method may be achieved by introducing one copy or two copies of polynucleotides encoding the polypeptide into the chromosome of a host cell. The introduction into the chromosome may be performed by introducing a vector, which is able to insert the polynucleotide into the chromosome of a host cell, into the host cell, but is not limited thereto. The vector is as described above.

The 2) method of modifying the expression regulatory sequence of a gene encoding TREK1 may include replacing the expression regulatory region (or expression regulatory sequence) which is operably linked to the expression cassette containing the gene encoding TREK1, vector, or the gene encoding TREK1 on the chromosome with a sequence having a strong activity. For example, the method may be achieved by inducing a modification on the sequence through deletion, insertion, non-conservative or conservative substitution or a combination thereof to further enhance the activity of the expression regulatory sequence or by replacing the sequence with a sequence having a stronger activity. The expression regulatory sequence may include, but is not particularly limited to, a promoter, an operator sequence, a sequence encoding a ribosome-binding site, and a sequence regulating the termination of transcription and translation, *etc.* Further, the modification may include a modification of inserting an enhancer region.

The 3) method of modifying the nucleotide sequence of the 5'-UTR or 3'-UTR of the gene transcript encoding TREK1 may be achieved by, for example, modifying the UTR sequence to be optimized for expression, but is not limited thereto.

The 4) method of modifying the amino acid sequence or the polynucleotide sequence may be achieved by inducing a modification on the sequence through deletion, insertion, non-conservative or conservative substitution of the amino acid sequence of the polypeptide or the polynucleotide sequence encoding the polypeptide, or a combination thereof to enhance the activity of the polypeptide, or by replacing the sequence with an amino acid sequence or polynucleotide sequence modified to have a stronger activity, or an amino acid sequence or polynucleotide sequence modified to enhance the activity. The modification of properly inserting an intron sequence or modification to include a poly A tail may also be included in the modification of the polynucleotide sequence, but is not limited thereto.

The 5) method of regulating the transcription or translation such that the activity of TREK1 is enhanced may include attaching a sequence such as an IRES onto the RNA sequence to promote and activate translation initiation, adding a translation enhancer, adding PABP to the poly A tail end, or including a Kozak sequence optimized for expression or a modified form thereof, *etc.* The method may also include introducing factors that facilitate ribosome initiation and elongation, or introducing histone modifying enzymes in combination to rearrange the structure of chromatin so that transcription can occur smoothly.

The 6) method (not part of the invention) of introducing a foreign polynucleotide exhibiting the activity of TREK1 may be achieved by introducing a foreign polynucleotide encoding a polypeptide that exhibits the same/similar activity to that of the polypeptide. The foreign polynucleotide is not limited by its origin or sequence as long as it exhibits the same/similar activity as TREK1.

The 7) method of codon optimization of the polynucleotide encoding TREK1 may be achieved by codon optimization of an endogenous polynucleotide to increase the transcription or translation, or by optimizing the codons thereof such that the optimized transcription and translation of the foreign polynucleotide can be achieved within the cell. For example, among codons present in the coding sequence of TREK1, it can be modified to replace codons rarely expressed in mammals or primates with codons frequently expressed in primates.

The 8) method of post-translational modification of TREK1 may include a modification or chemical modification to enhance the activity of TREK1, for example, it may be modifying the transmembrane domain or C-terminus of TREK1 so that TREK1 is in an open form. Examples of such modifications include modifications such as phosphorylation or dephosphorylation, and one example includes dephosphorylation of the Ser333 residue at the C-terminus. However, the modification is not limited to the above-described examples.

The method of the present invention may include administering the pharmaceutical composition including the TREK1 activator in a pharmaceutically effective amount (not part of the invention).

An appropriate total daily dose of the pharmaceutical composition may be determined within the scope of correct medical judgment by a practitioner, and the pharmaceutical composition may be administered once or several times in divided doses. However, for the purpose of the present invention, it is preferred that the specific therapeutically effective dose of the pharmaceutical composition for any particular patient be applied differently depending on the kind and degree of responses to be achieved, specific compositions including whether other agents are occasionally used therewith, the patient's age, body weight, health conditions, sex and diet, administration time, administration route, excretion rate of the composition, duration of treatment, various factors including drugs used in combination or simultaneously with the specific compositions, and similar factors well known in the medical field.

In one example, when the TREK1 activator of the present invention includes an expression cassette or vector, viral particles in any concentration necessary for effective transduction can be prepared for contact with preadipocytes *in vitro* or *in vivo.* In one example, the unit dose of the pharmaceutical composition may be determined using multiplicity of infection (MOI). MOI refers to the ratio or fold of a vector or viral genome to cells to which a nucleic acid can be delivered.

In any one of the above-described embodiments, the subject to which the method of the present invention can be applied refers to mammals including humans, rats, livestock, *etc.* that have or can develop the disease, but any subject that can be treated with the regulation of TREK1 activity of the present invention are included without limitation.

As used herein, the term "weakening" is a comprehensive concept including both reduced or no activity compared to its endogenous activity. The weakening may be used interchangeably with terms such as inactivation, deficiency, down-regulation, decrease, reduce, attenuation, *etc.*

The weakening may also include a case where the protein activity itself is decreased or removed compared to the activity of the endogenous protein due to a mutation of the polynucleotide encoding the protein; a case where the overall level of protein activity and/or concentration (expression level) in the cells/tissues is decreased due to the inhibition of expression of the gene encoding the same, or the inhibition of translation into the protein; a case where the polynucleotide is not expressed at all; and/or a case where no protein activity is observed even when the polynucleotide is expressed. As used herein, the "endogenous activity" refers to the activity of a particular protein originally possessed by wild-type or a non-modified cells, tissues, or subjects before transformation, when a trait is altered by natural (such as introduction of an activator or inactivator) or artificial factors.

In one embodiment, the weakening of TREK1 may be achieved by:
1) deleting a part or all of the gene encoding TREK1;
2) modifying the expression regulatory region such that the expression of the gene encoding TREK1 is decreased;
3) modifying the amino acid sequence constituting the protein or the polynucleotide encoding the same such that the activity of TREK1 is removed or weakened;
4) post-translational modification such that the activity of TREK1 is removed or weakened;
5) modifying the nucleotide sequence encoding the 5'-UTR or 3'-UTR of the gene transcript encoding the protein such that the activity of TREK1 is removed or weakened;
6) introducing an antisense oligonucleotide which binds complementarily to the gene transcript encoding TREK1;
7) inhibiting the transcription or translation of the protein such that the activity of TREK1 is removed or weakened;
8) adding a promoter, which is to be reversely transcribed on the 3' terminus of the open reading frame (ORF) of the gene sequence encoding TREK1 (Reverse transcription engineering, RTE);
9) treating a TREK1 activity inhibitor;
10) a combination of two or more selected from the methods 1) to 9) above, but is not particularly limited thereto.

For example,
the 1) method of deleting a part or all of the gene encoding TREK1 may be achieved by knocking out all of the polynucleotide encoding the endogenous TREK1 within the chromosome, or by replacing the polynucleotide with a polynucleotide having a partially deleted nucleotide, or with a marker gene. In another example, the polynucleotide sequence may be modified or deleted using gene scissors, *etc.* As for the gene scissors, zinc finger nuclease (ZFN), transcription activator-like effector nuclease (TALEN), clustered regularly interspaced short palindromic repeat (CRISPR), *etc.* are known, and these means can be used appropriately.

The 2) method of modifying the expression regulatory region (expression regulatory sequence) may be achieved by inducing a modification on the expression regulatory region (expression regulatory sequence) through deletion, insertion, non-conservative substitution or conservative substitution, or a combination thereof; or by replacing the sequence with a sequence having a weaker activity. The expression regulatory region may include a promoter, an operator sequence, a sequence encoding a ribosome-binding site, and a sequence for regulating the termination of transcription and translation, but is not limited thereto.

The 3) method of modifying the amino acid sequence or the polynucleotide sequence may be achieved by inducing a modification on the sequence through deletion, insertion, non-conservative or conservative substitution of the amino acid sequence or the polynucleotide sequence encoding the same, or a combination thereof to weaken the activity of TREK1, or by replacing the sequence with an amino acid sequence or a polynucleotide sequence modified to have a weaker activity, or an amino acid sequence or a polynucleotide sequence modified to have no activity, but are not limited thereto. For example, the TREK1 activity may be inhibited or weakened by introducing a mutation into the polynucleotide sequence to form a termination codon, but is not limited thereto.

The 4) method of post-translational modification includes modification or chemical modification such that the activity of TREK1 is weakened or removed, for example, the transmembrane domain or the C-terminus may be modified to block TREK1. Example thereof may include a modification that phosphorylates the C-terminus. Additionally, it is known that when the C-terminal 333 Ser residue of TREK1 is phosphorylated, TREK1 is inactivated.

The 5) method of modifying the nucleotide sequence encoding the 5'-UTR or 3'-UTR of the gene transcript encoding TREK1 may be achieved, for example, by substituting the nucleotide sequence with another UTR sequence having a lower expression rate than the endogenous sequence, but is not limited thereto.

The 6) method of antisense oligonucleotides that binds complementarily to the transcript of the gene encoding TREK1 include, for example, siRNA, shRNA, miRNA, aptamer, *etc.* Such antisense nucleotides include both coding and noncoding RNA, and may include a sequence that is partially or completely complementary to the transcript of the gene whose activity is to be weakened. Antisense nucleotides can modulate their activity by participating in actions such as transcription conflicts, DNA methylation, histone modifications, or splicing, editing, and translation of mRNA.

The 7) method of inhibiting the transcription or translation of the protein such that the activity of TREK1 is removed or weakened may include modification on the Kozak sequence to prevent translation or to inhibit translation; modification on the TREK1 gene or expression cassette containing the TREK1 gene that cause poly A tail to be degraded, and histone modifications such as methylation and deacetylation. It may also include the introduction of factors that interfere with binding of tRNA or mRNA to ribosomes or inhibit transcription.

The 8) an antisense transcript may be formed by introduction of an antisense promoter. The antisense promoter may be located upstream, downstream or in an intergenic region of the sense transcription promoter. The antisense promoter may be located in the 5'-UTR, exon, intron, or 3'-UTR of the same gene as the sense promoter, or located in an adjacent gene or intergenic region. For example, when the antisense promoter is located in the 5'-UTR, transcription can be regulated by competing with the sense promoter and elements in the 5'-UTR.

The 9) TREK1 activity inhibitor can be used without limitation in its form, such as a compound, natural extract, chemical, nucleic acid, peptide, virus, or vector containing the nucleic acid, as long as it can target and inhibit the activity of TREK1. Such activity inhibitors may be, but are not limited to, ribozymes, DNAzymes, peptide nucleic acids (PNAs), peptides or antibodies. Examples of substances known to inhibit the activity of TREK1 are disclosed in Djillani, A., Mazella, J., Heurteaux, C., and Borsotto, M. (2019) Role of TREK-1 in Health and Disease, Focus on the Central Nervous System. Front. Pharmacol. 10:379, etc*.*

In one embodiment, the weakening may be weakening in preadipocyte cells.

It is further another aspect of the present invention to provide an in vitro method for screening a preventive or therapeutic agent for obesity, including:
(a) administering a candidate substance for preventing or treating obesity into preadipocyte cells isolated from a subject; and (b) measuring the level of TREK1 activity in the cells of step (a).

The TREK1 and obesity are the same as described above.

The screening method includes selecting the candidate substance as a substance for preventing or treating obesity when the TREK1 activity level is increased as a result of the measurement in step (b).

It is still further another aspect of the present invention to provide a method for regulating the differentiation of adipocytes, including regulating the TREK1 activity level (not part of the invention).

The method may include inhibiting differentiation into adipocytes by increasing TREK1 activity in preadipocyte cells and/or inducing differentiation into adipocytes by weakening TREK1 activity in preadipocyte cells. In one embodiment, the method may include inhibiting differentiation into adipocytes by increasing TREK1 activity in preadipocyte cells and/or inducing differentiation into adipocytes by weakening TREK1 activity in preadipocyte cells *in vitro* or *in vivo.* The TREK1 and regulation of its activity level is the same as described above.

### [Advantageous Effects]

As disclosed in the present invention, not only obesity can be prevented or treated by regulating the activity of TREK1, and it can be effectively used for the preparation of obesity-induced models, research on regulatory mechanism for obesity *in vivo,* and the development of therapeutic agents.

In addition, since TREK1 of the present invention is an ion channel protein, which is a membrane protein, it does not need to penetrate into the cells, and much safer and fewer side effects can be expected compared to the method that acts on the brain.

### [Brief Description of Drawings]

FIG. 1 is a schematic diagram (FIGS. 1B to 1E) showing a phenomenon in which the potassium conductivity of cells is significantly reduced and the cell membrane potential is depolarized in the process of differentiating the 3T3L1 cell line, which is a preadipocyte, into adipocytes (FIG. 1A).

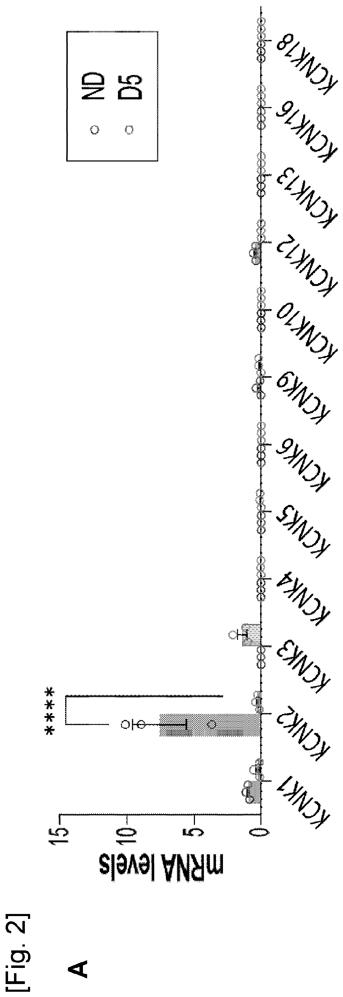

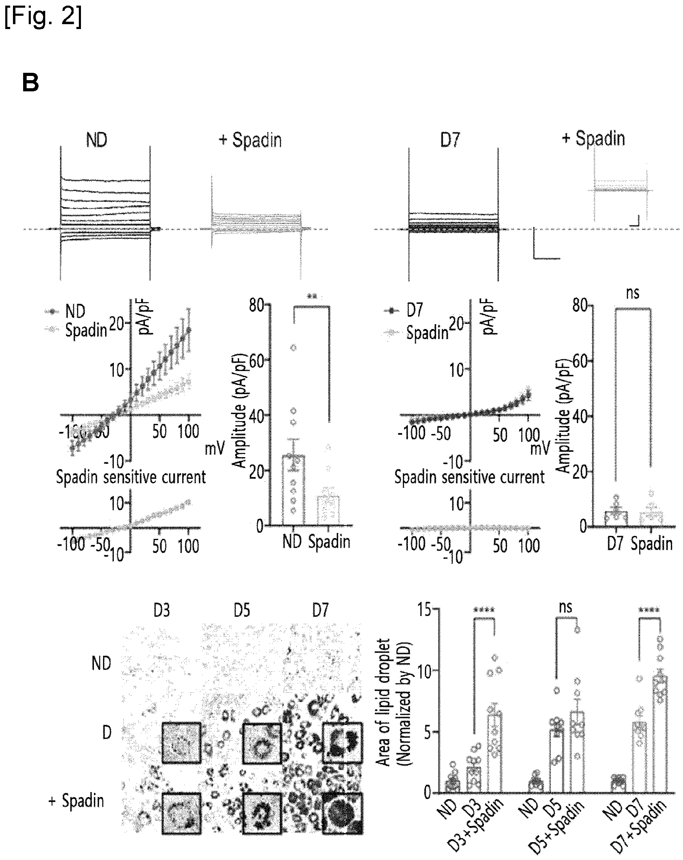

FIG. 2 confirmed the expression of 12 proteins with known functions among various K2P (two-pore potassium) ion channel proteins that maintain cell membrane potential, by comparing before and after inducing differentiation of the 3T3L1 cell line, which is a preadipocyte. Among these proteins, the expression of TREK1 is selectively very high and is specifically decreased in the differentiation process (FIG. 2A). Additionally, when spadin, known as a TREK1 inhibitor, was treated, the potassium conductivity of undifferentiated adipocytes was decreased and differentiation was further enhanced (FIG. 2B).
FIG. 3 is a diagram showing that when 3T3L1 cells, which are preadipocytes, are treated with spadin, a TREK1 inhibitor, intracellular calcium concentration may increase and voltage-dependent calcium ion channels are involved in this process.
FIG. 4 is a view showing that when the primary cultured adipocytes are treated with spadin, a TREK1 inhibitor, or mRNA is inhibited by treatment with TREK1 shRNA, potassium conductivity is decreased, membrane potential is increased, and differentiation into adipocytes is significantly increased.
FIG. 5 is a diagram showing that when a high-fat diet was performed using TREK1-knockout mice lacking TREK1 in order to confirm the importance of TREK1 for obesity at the individual level, the total body weight change in normal mice was not significant, but the weight of adipose tissue was significantly increased and the decomposition process of glucose was also significantly increased.
FIG. 6 is a diagram showing that the size of adipocytes was significantly increased in TREK1-knockout mice and further increased by a high-fat diet.
FIG. 7 is a diagram showing that when ML402, an activator of TREK1, was treated in the differentiation process of 3T3 cells, which are preadipocytes, the differentiation into adipocytes was significantly reduced in a concentration-dependent manner.

### [Detailed Description of Preferred Embodiments]

The present invention will be described in detail by way of Examples and Experimental Examples. However, these Examples and Experimental Examples are given for illustrative purposes only, and the scope of the present invention is not intended to be limited to or by these Examples and Experimental Examples.

### Example 1. Effect of TREK1 in differentiation process of 3T3L1 cells, which are preadipocytes

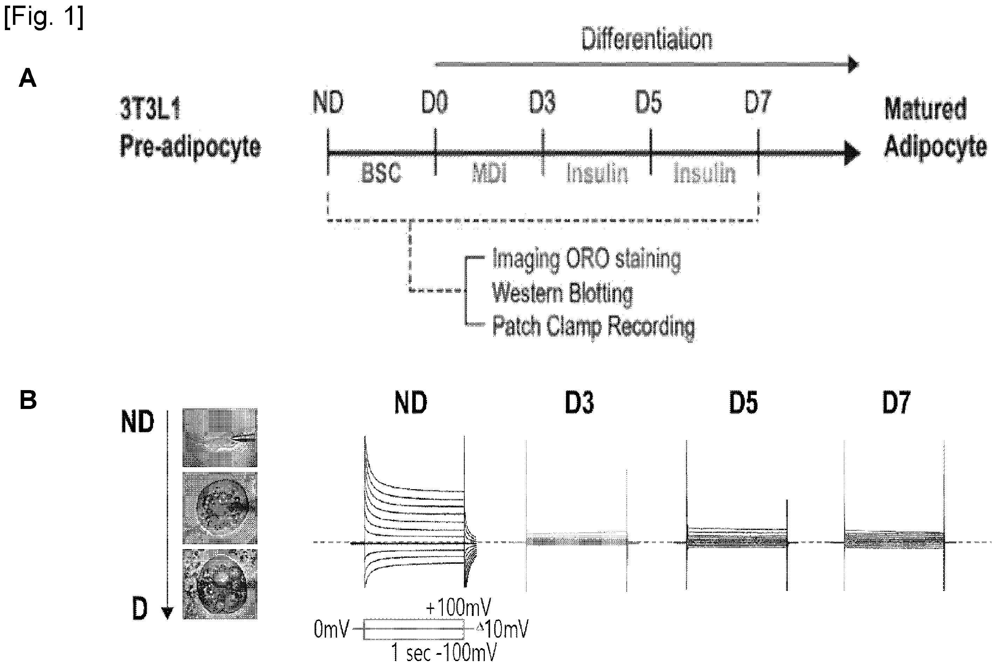

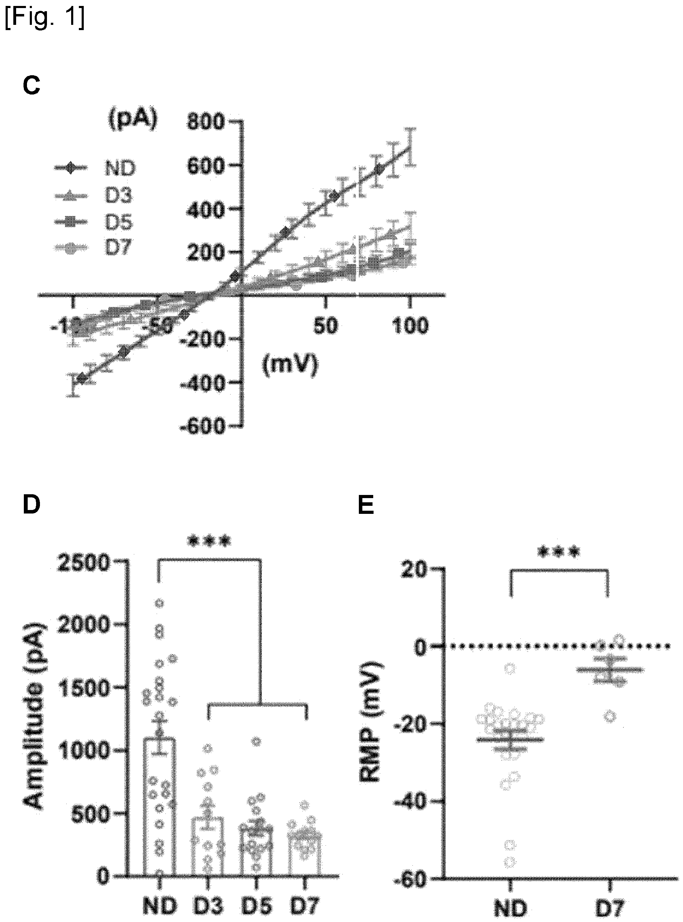

The 3T3L1 cell line, a preadipocyte cell line, was treated with MDI (Dexamethasone, IBMX, insulin) as a differentiation-inducing agent, and differentiation was induced for 7 days, and the differentiation into adipocytes was confirmed by staining with oil red O (FIG. 1A).

3T3-L1 cells were purchased from ATCC (American Type Culture Collection) and cultured in Dulbecco's Modified Eagle Medium (Gibco, NY, USA) with 10% (v/v) bovine serum (Gibco, NY, USA) and 1% v/v) penicillin/streptomycin (Thermo Fisher Scientific, NJ, USA). Cultures were maintained in a humidified 5% CO₂ incubator at 37°C. After reaching 80% confluence, the differentiation of 3T3L1 cells was induced by replacing the medium with DMEM containing 10% FBS plus 1 µg/mL insulin, 0.5 mM isobutyl-1-methylxanthine (IBMX), 1 µM and 2 µM Rosiglitazone for 3 days. Afterward the medium was changed with DMEM containing 10% FBS plus 1 µg/mL insulin, and the cells were further cultured for 2 to 4 days. Culture medium contained a combination of IBMX, dexamethasone (Dex), insulin, rosiglitazone, and fetal bovine serum (FBS) to differentiate preadipocyte into mature adipocytes. After differentiation, experiments were conducted at three points. Each time the medium was changed over time, the cells were differentiated with IBMX medium for 3 days, and then changed to insulin medium every 2 days thereafter. As a result of confirmation by a microscope, the cell shape of 3T3-L1 was changed over time and intracellular lipid droplets were produced.

As a result of measuring the potassium ion selective conductivity by the patch clamp technique using a cell line model in which differentiation into adipocytes was well confirmed, a sharp decrease in potassium ion conductivity was confirmed from the 3rd day, the initial stage of differentiation (FIGS. 1B to D). In addition, it was confirmed that the resting membrane potential (RMP) was highly depolarized in this process (FIG. 1E). From this result, the relevance of the K2P ion channel protein, which is known to be involved in the resting membrane potential among various potassium ion channel proteins, could be expected.

In the 3T3L1 cell line, mRNA was extracted before and after induction of differentiation to confirm the expression patterns of 12 K2P proteins whose ion channel activity have been reported. Primers reported in Supplementary Table 1 of Mi Hwang, E., Kim, E., Yarishkin, O. et al. A disulphide-linked heterodimer of TWIK-1 and TREK-1 mediates passive conductance in astrocytes. Nat. Commun. 5:3227 (2014) (https://doi.org/10.1038/ncomms4227) were used.

Interestingly, it was confirmed that the expression of TREK1 among the K2P proteins was selectively very high and rapidly decreased after differentiation was induced (FIG. 2A). These results were not observed in other K2P proteins, but when spadin, which is known to act very selectively on TREK1, is treated to 3T3L1 cell line, it was confirmed that the conductivity was generated by the TREK1 protein, as most of the potassium conductivity was decreased in the undifferentiated condition (FIG. 2B). In addition, it was confirmed that when spadin was treated in the process of differentiation into adipocytes, the degree of differentiation was significantly increased (FIG. 2B).

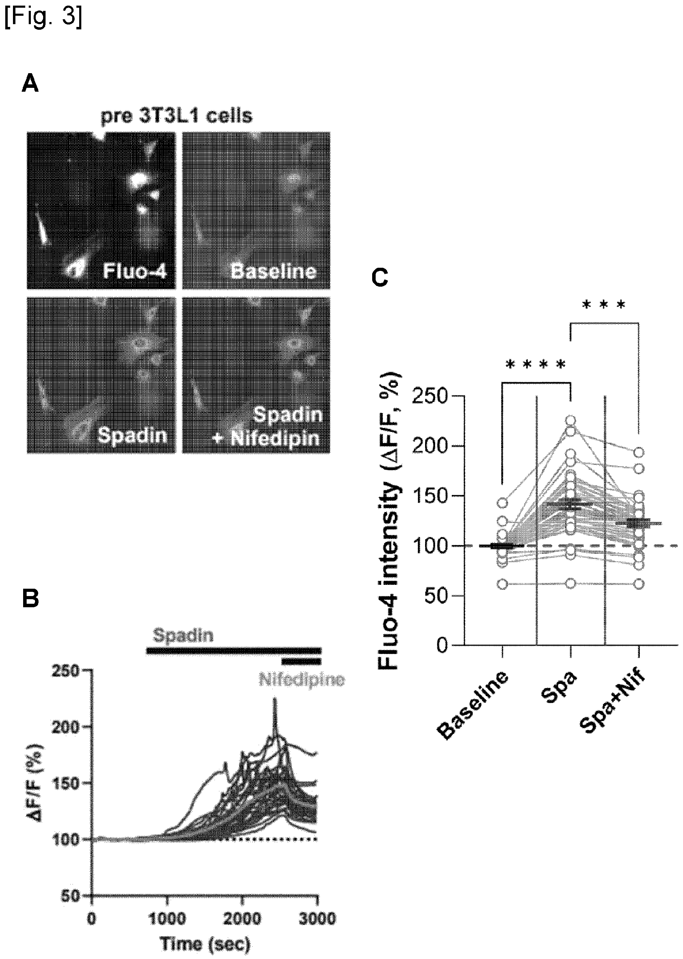

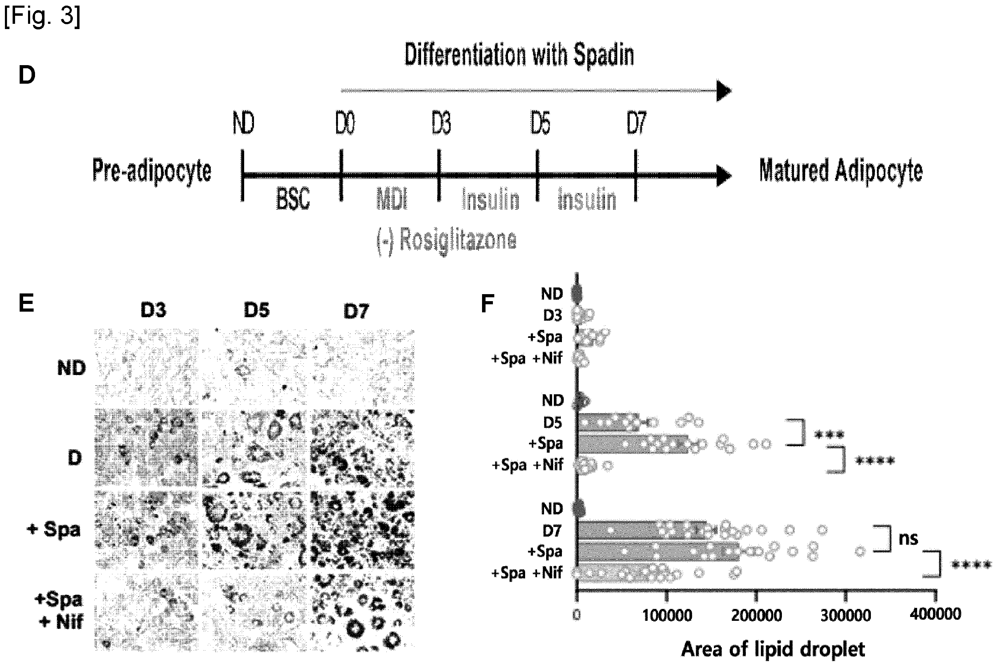

As previously known, the process of differentiation into adipocytes must be accompanied by an increase in calcium ion concentration, and as a result of treatment with spadin, a TREK1 inhibitor, and measuring the intracellular calcium ion concentration, a significant increase in calcium ions was confirmed 30 minutes after spadin treatment (FIG. 3A). This increase in calcium ions was effectively inhibited by nifedifine, a voltage-dependent calcium ion channel inhibitor (FIGS. 3B and 3C), and it was confirmed that differentiation into adipocytes was also inhibited (FIGS. 3D and 3E). In summary, the activity of TREK1, which is a potassium ion channel, was decreased in the process of differentiation into adipocytes, and as a result, the cell membrane potential was depolarized, and voltage-dependent calcium ion channels were activated, resulting in increasing intracellular calcium ion concentration and promoting the differentiation of adipocytes.

### Example 2. Effect of TREK1 in primary cultured adipocytes

Since there is a possibility that the results shown in Example 1 may not reproduce the actual *in vivo* environment, a primary culture experiment was performed using adipocytes of mice.

The method for obtaining primary cultured adipocytes is as follows. Primary white adipocytes were isolated from inguinal WAT 6-8 week mouse (C57BL/6). The extracted WAT was washed in a 15 mL tube. The enzyme solution was mixed with collagenase Type II (Worthington Industries, Columbus, OH, USA) and PBS (Gibco, NY, USA), and the tissue was incubated in 20 mL enzyme solution in an incubator at 37°C for 1 hour. After filtering, the tissue was added to a new 50 mL tube and centrifuged at 10,000 rpm for 10 minutes. The adipocytes floating in the solution were transferred to a new tube using a pipette. Then, the adipocytes were once more centrifuged in DMEM/F12 (Gibco, NY, USA) containing 10% (v/v) FBS. After transferring the fatty layer to the cell culture flask, and the flask was filled with DMEM/F12 + mixture. The flask was inverted and incubated in a 37°C CO₂ incubator, and the medium was changed after about 5 days.

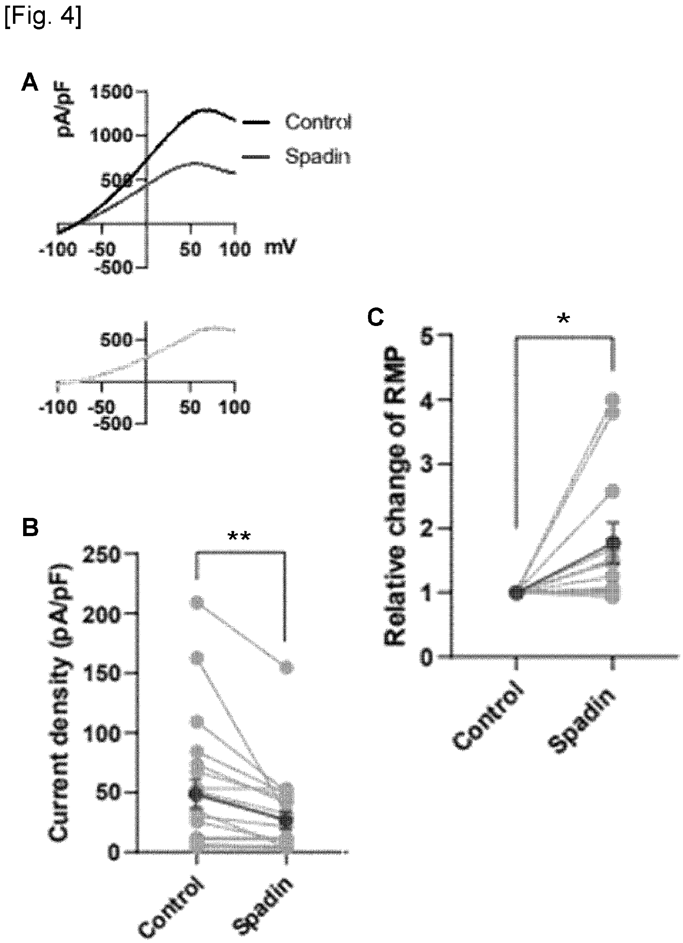

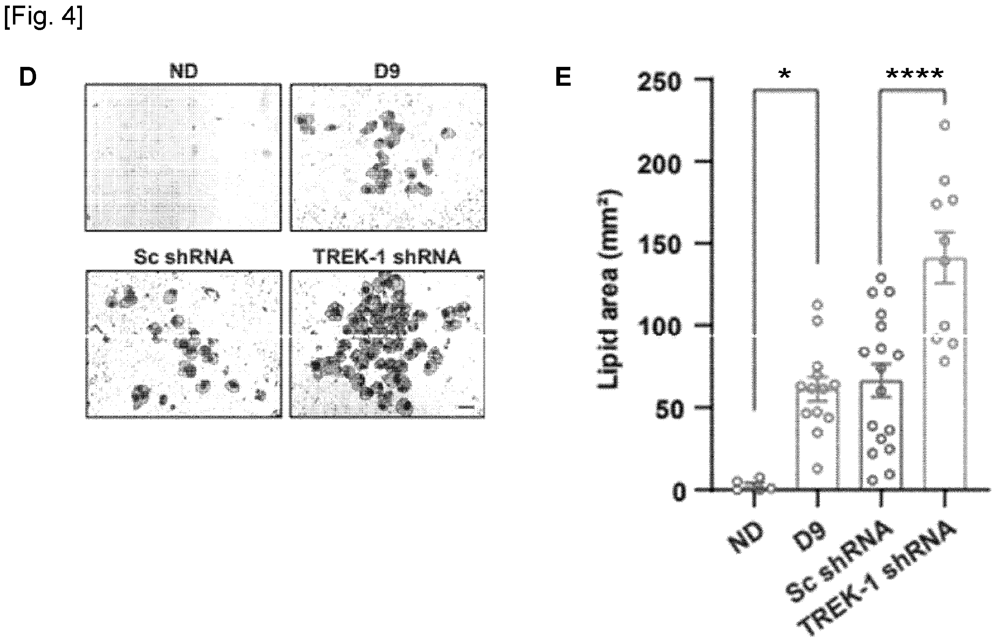

When the primary cultured adipocytes were treated with spadin (1 µM concentration), a TREK1 inhibitor, the potassium ion conductivity was significantly decreased (FIGS. 4A and 4B) as in the cell line result of Example 1, and the resting membrane potential was also increased (FIG. 4C).

In addition, differentiation was induced after treatment with TREK1 shRNA (gcgtggagatctacgacaagt, CDS 998-1018), which was verified well in previous studies, in order to confirm whether the same effect was exhibited by the reduction of TREK1 protein (see Hwang, E. M. et al. A disulphide-linked heterodimer of TWIK-1 and TREK-1 mediates passive conductance in astrocytes. Nat. Commun. 5:3227 (2014)). shRNA was used in an amount of 1 µg/µL. As a result, it was confirmed that the area of the differentiated adipocytes increased significantly compared to that treated with scrambled shRNA (FIG. 4E). This result shows that a decrease in TREK1 had an effect on the membrane potential and promoted the differentiation process in the process of differentiation of adipocytes in primary cultured mice, as in cell lines.

### Example 3. Effect on fat diet in TREK1-deficient mice

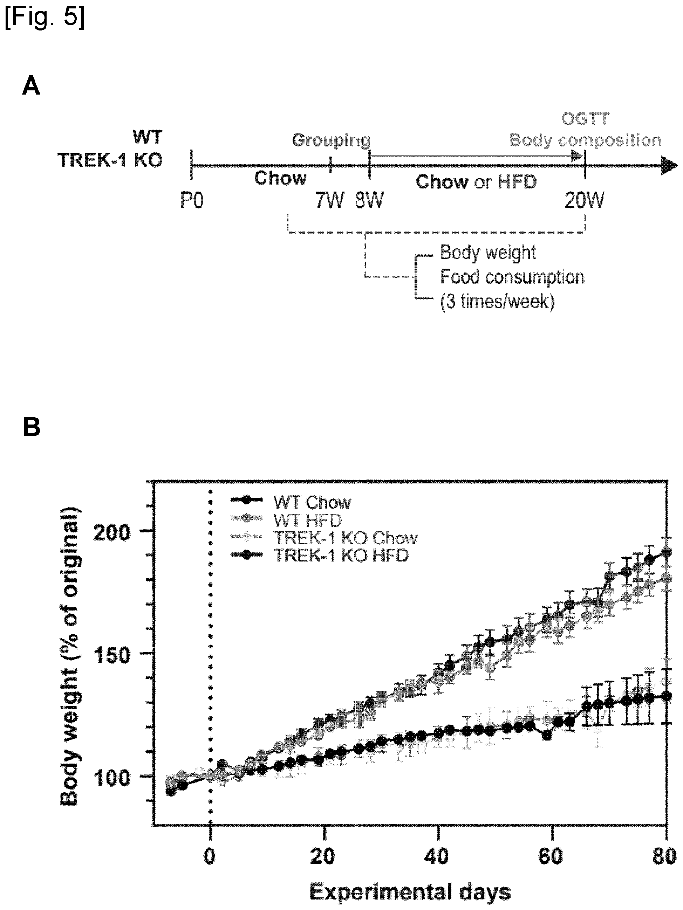

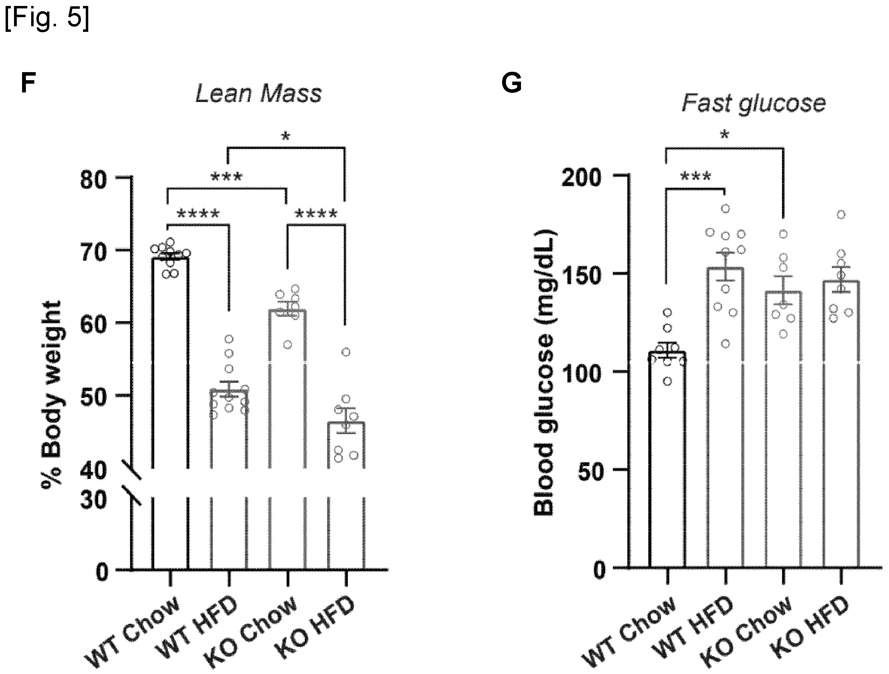

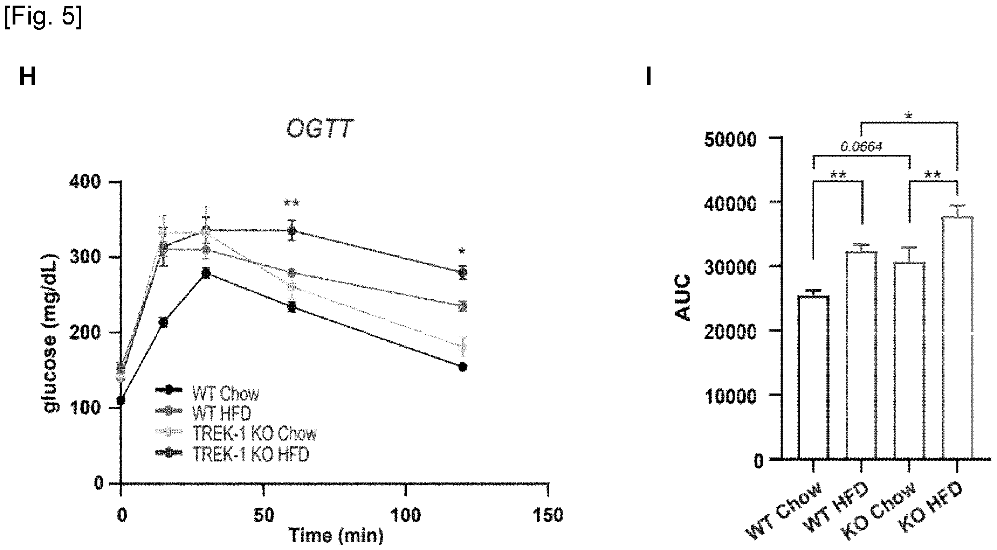

In order to confirm whether TREK1 was actually involved in the process of differentiation into adipocytes at the individual level, mice lacking only TREK1 were fed with a normal diet and a fat diet to confirm the effect on the obesity process (FIG. 5A).

TREK1-deficient mice were prepared by removing exon 4 after exon 2 including ATG using the CRISPR/Cas9 system. A feed with 10 kCal% fat content (Research Diets, D12450B) was fed to the normal diet group, and a feed with 60 kCal% fat content (Research Diets, D12492) was fed to the fat diet (HFD) group. The diet was continued for 12 weeks, and the amount of diet and weight were measured every 2 days.

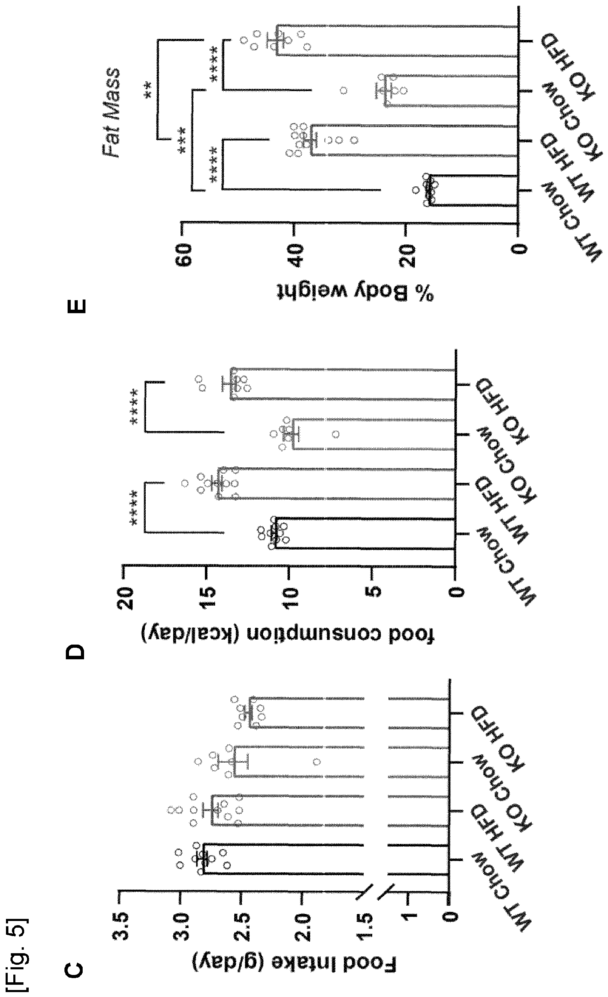

Although there was no significant difference in total body weight in normal mice and TREK1-deficient mice during the 12-week diet (FIG. 5B), and there was no significant difference in intake (FIGS. 5C and 5D), it was confirmed that TREK1-deficient mice significantly increased the actual fat weight even by the normal diet (FIG. 5E). The TREK1-deficient mice increased their fat weight more than normal mice by way of the fat diet, and it was confirmed that the degradation efficiency was significantly reduced when glucose was injected after a 12-hour fast (FIGS. 5G to 5I).

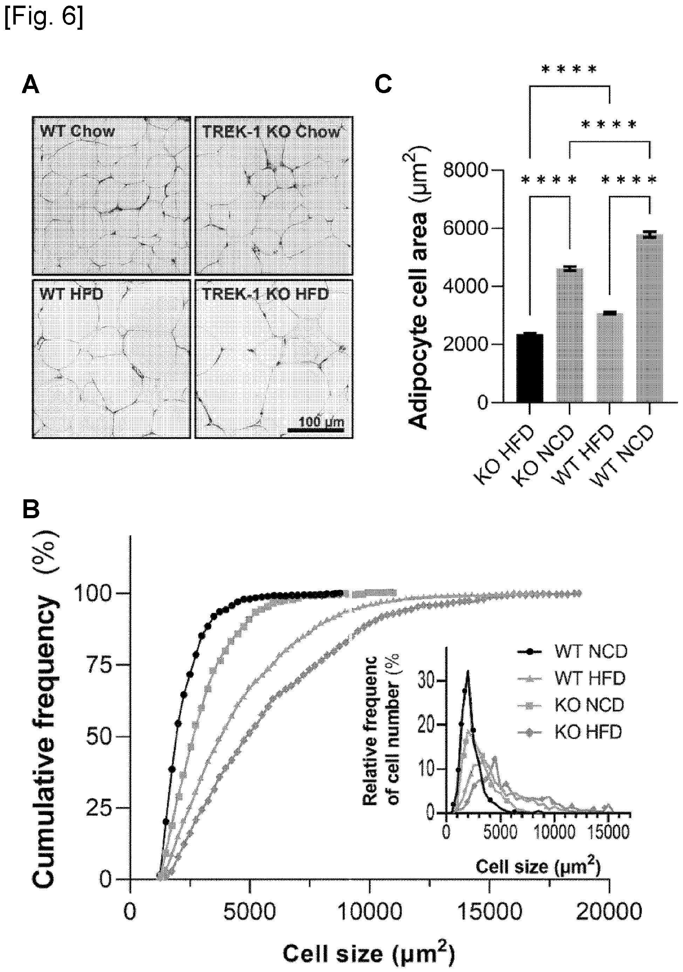

When the actual size and number of adipocytes were confirmed using a microscope, the number of adipocytes in TREK1-deficient mice was significantly increased compared to normal mice, and it was confirmed that the size was also increased (FIG. 6A). In addition, it was confirmed that the change was further increased by the fat diet (FIGS. 6B and 6C).

Based on the results, it was confirmed that inhibiting the activity of TREK1 induced obesity in both the normal diet and the fat diet groups.

### Example 4. Adipocyte differentiation inhibitory effect on TREK1 activator

Since it was confirmed through Examples 1, 2, and 3 that selective inhibition of TREK1 was essential for the differentiation process of adipocytes, it was confirmed whether treatment with an activator of TREK1 could effectively inhibit the differentiation process.

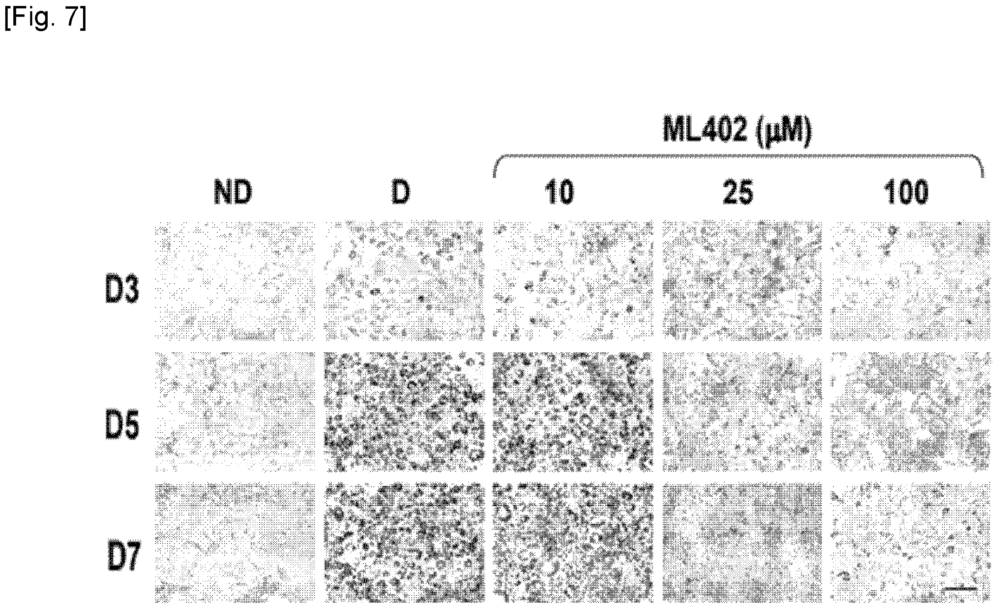

The 3T3L1 cell line, in which the same effect as *in vivo* and conditions was well confirmed, was treated with ML402, which has been reported to have an active effect on TREK1. Specifically, the cells were sampled on the 3rd, 5th, and 7th days by treating ML402 together from the time of differentiation at concentrations of 10 µM, 25 µM, and 100 µM, respectively. As a result of examining the effect on the differentiation process, the amount of triglycerides did not show a significant difference on the 3rd day, as compared with the control group, but decreased in a concentration-dependent manner on the 5th and 7th days, thereby confirming that the differentiation process was inhibited in a concentration-dependent manner as expected (FIG. 7).

Based on the results, it was confirmed that obesity can be suppressed by enhancing TREK1 activity.

The exemplary embodiments disclosed herein are only for illustrative purposes and should not be construed as limiting the scope of the present invention. The scope of the present invention is therefore indicated by the appended claims rather than by the foregoing description.

## Claims

1. A pharmaceutical composition for use in preventing or treating obesity, comprising a potassium two pore domain channel subfamily K member 2 (TREK1) activator, which is a substance that increases the activity of TREK1 protein and/or the expression of a gene encoding TREK1 protein, as an active ingredient.

2. The pharmaceutical composition for use of claim 1, wherein the TREK1 activator is selected from riluzole, lithium, gabapentin, valproate, carbamazepine, ML67, ML67-33, ML335, ML402, arachidonic acid, chloroform, halothane, isoflurane, diethyl ether, fenamate, flufenamic acid, BL-1249, RNE28, GI-530159, PUFA and/or pharmaceutically acceptable salts thereof.

3. The pharmaceutical composition for use of claim 1 or 2, wherein the TREK1 activator is a vector containing cDNA of the gene encoding TREK1.

4. A composition for use in a method for preventing or treating obesity, the composition comprising a TREK1 activator as active ingredient, the method comprising enhancing the activity of potassium two pore domain channel subfamily K member 2 (TREK1), wherein the enhancement of the TREK1 activity includes a treatment with a TREK1 activator and wherein the enhancement of TREK1 is achieved by:
1) increasing the intracellular copy number of a polynucleotide encoding TREK1;
2) modifying the expression regulatory region of a gene encoding TREK1 with a sequence having a strong activity;
3) modifying the nucleotide sequence encoding the 5'-UTR or 3'-UTR of the gene transcript encoding TREK1;
4) modifying the amino acid sequence or the polynucleotide sequence encoding the same such that the activity of TREK1 is enhanced;
5) regulating the transcription or translation such that the activity of TREK1 is enhanced;
6) codon optimization of the polynucleotide encoding TREK1;
7) post-translational modification such that the activity of TREK1 is enhanced; or
8) a combination of two or more selected from above 1) to 7).

5. The composition for use according to claim 4, wherein the enhancement of the TREK1 activity is achieved by increasing TREK1 activity in preadipocytes.

6. An in vitro method for screening a preventive or therapeutic agent for obesity, comprising:
(a) administering a candidate substance for preventing or treating obesity into preadipocyte cells isolated from a subject; and (b) measuring the level of potassium two pore domain channel subfamily K member 2 (TREK1) activity in the cells of step (a); and (c) selecting the candidate substance as a substance for preventing or treating obesity, when the TREK1 activity level is increased as a result of the measurement in step (b).

## Patentansprüche

1. Pharmazeutische Zusammensetzung zur Verwendung bei der Vorbeugung oder Behandlung von Adipositas, umfassend einen Aktivator des Proteins TREK1 (Zwei-Porendomänen-Kaliumkanal, Unterfamilie K, Mitglied 2), bei dem es sich um eine Substanz handelt, welche die Aktivität von TREK1-Protein und/oder die Expression eines für TREK1-Protein kodierenden Gens erhöht, als Wirkstoff.

2. Pharmazeutische Zusammensetzung zur Verwendung nach Anspruch 1, wobei der TREK1-Aktivator aus Riluzol, Lithium, Gabapentin, Valproat, Carbamazepin, ML67, ML67-33, ML335, ML402, Arachidonsäure, Chloroform, Halothan, Isofluran, Diethylether, Fenamat, Flufenaminsäure, BL-1249, RNE28, GI-530159, PUFA und/oder deren pharmazeutisch verträglichen Salzen ausgewählt ist.

3. Pharmazeutische Zusammensetzung zur Verwendung nach Anspruch 1 oder 2, wobei der TREK1-Aktivator ein Vektor ist, der cDNA des für TREK1 kodierenden Gens enthält.

4. Zusammensetzung zur Verwendung in einem Verfahren zur Vorbeugung oder Behandlung von Adipositas, wobei die Zusammensetzung einen TREK1-Aktivator als Wirkstoff umfasst, wobei das Verfahren das Steigern der Aktivität des Proteins Zwei-Porendomänen-Kaliumkanal, Unterfamilie K, Mitglied 2 (TREK1) umfasst, wobei die Steigerung der TREK1-Aktivität eine Behandlung mit einem TREK1-Aktivator umfasst und wobei die Steigerung von TREK1 erreicht wird durch:
1) Erhöhen der intrazellulären Kopienzahl eines Polynukleotids, das für TREK1 kodiert;
2) Modifizieren der Expressionsregulationsregion eines für TREK1 kodierenden Gens mit einer Sequenz, die eine starke Aktivität aufweist;
3) Modifizieren der Nukleotidsequenz, welche für die 5'-UTR oder die 3'-UTR des für TREK1 kodierenden Gentranskripts kodiert;
4) Modifizieren der Aminosäuresequenz oder der für selbige kodierenden Polynukleotidsequenz derart, dass die Aktivität von TREK1 gesteigert wird;
5) Regulieren der Transkription oder Translation derart, dass die Aktivität von TREK1 gesteigert wird;
6) Codon-Optimierung des für TREK1 kodierenden Polynukleotids;
7) posttranslationales Modifizieren derart, dass die Aktivität von TREK1 gesteigert wird; oder
8) eine Kombination aus zwei oder mehr aus den obigen Vorgängen 1) bis 7) ausgewählten Vorgängen.

5. Zusammensetzung zur Verwendung nach Anspruch 4, wobei die Steigerung der TREK1-Aktivität durch Erhöhung der TREK1-Aktivität in Präadipozyten erreicht wird.

6. In-vitro-Verfahren zum Screening eines präventiven oder therapeutischen Mittels gegen Adipositas, umfassend:
(a) Verabreichen einer Kandidatensubstanz zur Vorbeugung oder Behandlung von Adipositas an aus einem Individuum isolierte Präadipozyten; und (b) Messen des Aktivitätsniveaus des Proteins Zwei-Porendomänen-Kaliumkanal, Unterfamilie K, Mitglied 2 (TREK1) in den Zellen aus Schritt (a); und (c) Auswählen der Kandidatensubstanz als Substanz zur Vorbeugung oder Behandlung von Adipositas, wenn das Ergebnis der Messung in Schritt (b) ein erhöhtes TREK1-Aktivitätsniveau zeigt.

## Revendications

1. Composition pharmaceutique destinée à être utilisée pour prévenir ou traiter l'obésité, comprenant, en tant qu'ingrédient actif, un activateur du membre 2 de la sous-famille K des canaux potassiques à deux domaines pore (TREK1), qui est une substance augmentant l'activité de la protéine TREK1 et/ou l'expression d'un gène codant pour la protéine TREK1.

2. Composition pharmaceutique selon la revendication 1, dans laquelle l'activateur de TREK1 est choisi parmi le riluzole, le lithium, la gabapentine, le valproate, la carbamazépine, le ML67, le ML67-33, le ML335, le ML402, l'acide arachidonique, le chloroforme, l'halothane, l'isoflurane, l'éther diéthylique, le fénamate, l'acide flufénamique, le BL-1249, le RNE28, le GI-530159, les AGPI et/ou leurs sels pharmaceutiquement acceptables.

3. Composition pharmaceutique destinée à l'utilisation selon la revendication 1 ou 2, dans laquelle l'activateur de TREK1 est un vecteur contenant de l'ADNc du gène codant pour TREK1.

4. Composition destinée à être utilisée dans un procédé de prévention ou de traitement de l'obésité, la composition comprenant un activateur de TREK1 en tant qu'ingrédient actif, le procédé comprenant l'étape consistant à augmenter l'activité du membre 2 de la sous-famille K des canaux potassiques à deux domaines pore (TREK1), dans lequel l'augmentation de l'activité de TREK1 comprend un traitement avec un activateur de TREK1 et dans lequel l'augmentation de TREK1 est atteinte par :
1) l'augmentation du nombre de copies intracellulaires d'un polynucléotide codant pour TREK1 ;
2) la modification de la région régulatrice de l'expression d'un gène codant pour TREK1 par une séquence présentant une forte activité ;
3) la modification de la séquence nucléotidique codant pour l'UTR 5' ou l'UTR 3' du transcrit du gène codant pour TREK1 ;
4) la modification de la séquence d'acides aminés ou de la séquence polynucléotidique codant pour celle-ci de telle sorte que l'activité de TREK1 soit augmentée ;
5) la régulation de la transcription ou de la traduction de telle sorte que l'activité de TREK1 soit augmentée ;
6) l'optimisation des codons du polynucléotide codant pour TREK1 ;
7) la modification post-traductionnelle de telle sorte que l'activité de TREK1 soit augmentée ; ou
8) une combinaison de deux ou plusieurs processus choisis parmi les processus 1) à 7) ci-dessus.

5. Composition destinée à l'utilisation selon la revendication 4, dans laquelle l'augmentation de l'activité de TREK1 est atteinte en augmentant l'activité de TREK1 dans des préadipocytes.

6. Procédé *in vitro* de criblage d'un agent préventif ou thérapeutique contre l'obésité, comprenant les étapes consistant à :
(a) administrer une substance candidate pour prévenir ou traiter l'obésité dans des cellules préadipocytaires isolées d'un sujet ; et (b) mesurer le niveau d'activité du membre 2 de la sous-famille K des canaux potassiques à deux domaines pore (TREK1) dans les cellules de l'étape (a) ; et (c) sélectionner la substance candidate en tant que substance pour prévenir ou à traiter l'obésité si le résultat de la mesure de l'étape (b) démontre que le niveau d'activité de TREK1 est augmenté.
